# EUROPEAN PATENT APPLICATION

(11) **EP 2 280 052 A1**
(43) Date of publication of application: **02.02.2011**
(21) Application number: 09728879.9
(22) Date of filing: 30.03.2009
(51) Int. Cl.: C09K 11/06, A61K 49/00, A61K 31/66, A61K 31/69, G01N 33/50

(54) **FLUORESCENT COMPOUNDS FOR THE DIAGNOSIS OF INFECTIONS, PRODUCTION METHOD, AND APPLICATIONS THEREOF**

(30) Priority: 04.04.2008 ES 200800951
(71) Applicant: Consejo Superior De Investigaciones Científicas, 28006 Madrid (ES); Universidad de Valladolid, Plaza Santa Cruz, 8 47002 Valladolid (ES); Universidad San Pablo Ceu, 28003 Madrid (ES)
(72) Inventor: ACUÑA FERNÁNDEZ, A. Ulises, 28006 Madrid (ES); AMAT GUERRI, Francisco, 28006 Madrid (ES); CARRILLO GALLEGO, Eugenia, 28040 Madrid (ES); HORNILLOS GOMEZ RECUERO, Valentín, 28006 Madrid (ES); MARCOS CELESTINO, Susana, 28006 Madrid (ES); REQUEJO ISIDRO, José María, 28006 Madrid (ES); RIVAS LÓPEZ, Luis Ignacio, 28040 Madrid (ES); SAUGAR CRUZ, José María, 28040 Madrid (ES); DEL AGUILA DE LA PUENTE, Carmen, 28003 Madrid (ES); MERAYO LLOVES, Jesús, 47011 Valladolid (ES)
(74) Representative: Pons Ariño, Angel
(86) International application number: PCT/ES2009/070077
(87) International publication number: WO 2009/121993

(57) **Abstract**

The invention relates to novel fluorescent compounds analogous to alkyl phospholipids, containing highly photostable fluorescent groups in the structure thereof and emitting in the visible spectrum wavelength range. Said compounds are easily introduced into micro-organisms, enabling the detection and identification of said organisms by means of fluorescence microscopy. The novel fluorescent compounds can also diagnose, rapidly and easily, the presence of pathogenic micro-organisms **characterised by** their resistance to treatment with alkyl phospholipids.

## Description

### FIELD OF THE TECHNIQUE

Pharmaceutical compositions for the clinical diagnosis of infectious diseases, preferably ocular, and to identify the presence of microorganisms.

### STATE OF THE TECHNIQUE

The correct treatment and prevention of certain diseases affecting the eye requires identification of a large variety of microorganisms capable of producing infections in said organ. Many of these eye infections can have severe consequences on the infected patient's vision, unless diagnosed and treated early. To this effect, in some cases it is necessary to detect the agent of infection directly in the infected organ, by extracting from the eye representative fragments of the tissue susceptible to infection (biopsy) or by analysing physiological fluids in direct contact with said tissues (tears, in this case). Usual methods for detecting microorganisms in the cited samples are based, frequently, on cultures to increase the population of the potential pathogenic microorganism, in order to facilitate its subsequent identification. These cultures may require extended periods of time, of 1-4 days, during which the patient cannot be treated with specific medication for the infection, given that the causing agent thereof is unknown. These extended waiting times can have extremely severe consequences for the infected organ, meaning that it would be enormously useful to have alternative methods of detection that considerably reduce the time required to make a correct diagnosis.

On a separate note, to prevent this type of infection it is very important for those elements that may eventually be in intimate contact with the eye during long periods of time, such as contact lenses and their corresponding cleaning fluids, to be conserved and used in high conditions of hygiene that ensure their sterility. As in the description above, in order to be able to obtain reliable information regarding the potential contamination of said eye prostheses and the conservation means thereof, it is frequently necessary to resort to laborious methods of microorganism culture. The complexity of current diagnosis techniques for these materials, based on cell cultures, limit in this case both the frequency as well as the extension of their application, with the ensuing increased risk of infection for users.

At the same time, alternative techniques for detecting infectious microorganisms of the fluorometric type have been developed, based on the use of a fluorescent marker compound that is added directly to the medium in which the microorganism's existence is suspected. If the fluorescent marker compound is incorporated into the infectious microorganism, it is possible to determine the presence and morphology thereof using various fluorescence display devices, such as microscopes or flow cytometers (Kawamoto, F., Rapid diagnosis of malaria by fluorescence microscopy with light microscope and interference filter. Lancet 1991, 337, 200-202). Since these devices allow very minor fluorescence intensities to be detected, in principle it is possible to carry out a correct diagnosis with a very small number of cells, considerably reducing the time required for this operation. Unfortunately, the use of this direct method of fluorescent marking for detecting eye infections is seriously restricted by the lack of specificity of fluorescent marking, caused because the affinity of traditional fluorescent markers for numerous pathogenic microorganisms of ophthalmological interest is very similar to the affinity for healthy eye cells and tissues, making it difficult to discriminate between the fluorescence corresponding to the pathogenic organisms.

The object of the present invention consists precisely of using a new simple and economic method that makes it possible to increase the affinity of the fluorescent marker for the infectious organism. The new method is based on using a fluorescent marker joined to a type of compounds with a relatively simple molecular structure, which shows much more affinity for the pathogenic organism than for the normal healthy eye cells. These compounds, used in the present invention for the selective incorporation of the fluorescent marker in the pathogenic organism, belong to the family of alkyl phospholipids, among which are found for example miltefosine (MT), edelfosine (ET) and ilmofosine (IM) (Croft, S. L.; Engel, J., Miltefosine--discovery of the antileishmanial activity of phospholipid derivatives. Trans R Soc Trop Med Hyg 2006, 100 Suppl 1, S4-8).

Miltefosine (MT) is currently used in the treatment of cutaneous breast cancer metastases, and is commercialised under the name MILTEX. Also, miltefosine demonstrates a potent antiparasitic activity, meaning that it is used in the treatment of human leishmaniasis (Soto, J.; Soto, P., Miltefosine: oral treatment of leishmaniasis. Expert Rev Anti Infect Ther 2006, 4, (2), 177-85) traded under the name IMPAVIDO (Zentaris S. A.) in Colombia, India and Germany. Recently, the use of MT has also been initiated for the treatment of canine leishmaniasis, using for this purpose the commercial composition MILTEFORAN (Virbac). At the same time, it has been observed that the cytotoxic activity of MT extends to other protozoa, such as *Trichomonas* (Blaha, C.; Duchene, M.; Aspock, H.; Walochnik, J., In vitro activity of hexadecylphosphocholine (miltefosine) against metronidazoleresistant and - susceptible strains of Trichomonas vaginalis. J Antimicrob Chemother 2006, 57, (2), 273-8), *Entamoeba histolytica* (Seifert, K.; Duchene, M.; Wernsdorfer, W. H.; Kollaritsch, H.; Scheiner, O.; Wiedermann, G.; Hottkowitz, T.; Eibl, H., Effects of miltefosine and other alkylphosphocholines on human intestinal parasite Entamoeba histolytica. Antimicrob Agents Chemother 2001, 45, (5), 1505-10), free living amoebae, such as *Acanthamoeba sp.* or *Naegleria sp.* (Schuster, F. L.; Guglielmo, B. J.; Visvesvara, G. S., In-vitro activity of miltefosine and voriconazole on clinical isolates of free-living amoebas: Balamuthia mandrillaris, Acanthamoeba spp., and Naegleria fowleri. J Eukaryot Microbiol 2006, 53, (2), 121-6; Walochnik, J.; Duchene, M.; Seifert, K.; Obwaller, A.; Hottkowitz, T.; Wiedermann, G.; Eibl, H.; Aspock, H., Cytotoxic activities of alkylphosphocholines against clinical isolates of Acanthamoeba spp. Antimicrob Agents Chemother 2002, 46, (3), 695-701) pathogenic for man as well as other human pathogenic microorganisms, including fungi, yeasts, (Obando, D.; Widmer, F.; Wright, L. C.; Sorrell, T. C.; Jolliffe, K. A., Synthesis, antifungal and antimicrobial activity of alkyl phospholipids. Bioorg Med Chem 2007, 15, (15), 5158-65; Widmer, F.; Wright, L. C.; Obando, D.; Handke, R.; Ganendren, R.; Ellis, D. H.; Sorrell, T. C., Hexadecylphosphocholine (miltefosine) has broad-spectrum fungicidal activity and is efficacious in a mouse model of cryptococcosis. Antimicrob Agents Chemother 2006, 50, (2), 414-21) and *Streptococcus pneumoniae* (Llull, D., Rivas, L., Garcia, E. In vitro bactericidal activity of the antiprotozoal drug miltefosine against Streptococcus pneumoniae and other pathogenic streptococci. Antimicrob Agents Chemother. 2007, 51, (5):1844-8).

Very little is known about the detailed mechanism whereby miltefosine produces its cytotoxic effects on the abovementioned organisms. The most recent studies in relation to certain specific parasites indicate that possibly said mechanism is of the multi-target type, given that the intracellular concentration of MT therein is very high, reaching in *Leishmania* values in the millimolar rank (Luque-Ortega, J. R.; Rivas, L., Miltefosine (hexadecylphosphocholine) inhibits cytochrome c oxidase in Leishmania donovani promastigotes. Antimicrob Agents Chemother 2007, 51, (4), 1327-32; Saugar, J. M.; Delgado, J.; Hornillos, V.; Luque-Ortega, J. R.; Amat-Guerri, F.; Acuna, A. U.; Rivas, L., Synthesis and Biological Evaluation of Fluorescent Leishmanicidal Analogues of Hexadecylphosphocholine (Miltefosine) as Probes of Antiparasite Mechanisms. J Med Chem 2007, 50, (24), 5994-6003). This hypothesis is also supported by the appearance of MT-resistant strains of *Leishmania,* characterised in that said resistance comes solely from functional defects in the specific translocators of the drug in the parasite (Seifert, K.; Perez-Victoria, F. J.; Stettler, M.; Sanchez-Canete, M. P.; Castanys, S.; Gamarro, F.; Croft, S. L., Inactivation of the miltefosine transporter, LdMT, causes miltefosine resistance that is conferred to the amastigote stage of Leishmania donovani and persists in vivo Int J Antimicrob Agents 2007, 30, (3), 229-35; Perez-Victoria, F. J.; Sanchez-Canete, M. P.; Seifert, K.; Croft, S. L.; Sundar, S.; Castanys, S.; Gamarro, F., Mechanisms of experimental resistance of Leishmania to miltefosine: Implications for clinical use. Drug Resist Updat 2006, 9, (1-2), 26-39; Perez-Victoria, F. J.; Sanchez-Canete, M. P.; Castanys, S.; Gamarro, F., Phospholipid translocation and miltefosine potency require both L. donovani miltefosine transporter and the new protein LdRos3 in Leishmania parasites. J Biol Chem 2006, 281, (33), 23766-75; Perez-Victoria, F. J.; Gamarro, F.; Ouellette, M.; Castanys, S., Functional cloning of the miltefosine transporter. A novel P-type phospholipid translocase from Leishmania involved in drug resistance. J Biol Chem 2003, 278, (50), 49965-71). These specific transporters belong to a family of amino phospholipid translocases; however, the precise identification of this transporter has only been carried out in *Leishmania donovani* (Perez-Victoria, F. J.; Gamarro, F.; Ouellette, M.; Castanys, S., Functional cloning of the miltefosine transporter. A novel P-type phospholipid translocase from Leishmania involved in drug resistance. J Biol Chem 2003, 278, (50), 49965-71), as LdMT3, and in *Saccharomyces cerevisiae* as Lem3 (Hanson, P. K.; Malone, L.; Birchmore, J. L.; Nichols, J. W., Lem3p is essential for the uptake and potency of alkylphosphocholine drugs, edelfosine and miltefosine. J Biol Chem 2003, 278, (38), 36041-50). In *Leishmania,* the mutations of the transporter entail the appearance of resistance to miltefosine and edelfosine, progressing with a concomitant inhibition of the incorporation of radioactive miltefosine into the parasite; however, in the case of loss of function of Lem3 of *S. cerevisiae,* the incorporation of the fluorescent phospholipid NBD-PC appears inhibited, associated to an edelfosine-resistant phenotype, which is due to a defective incorporation of the drug, although said effect has not been measured experimentally (Hanson, P. K.; Malone, L.; Birchmore, J. L.; Nichols, J. W., Lem3p is essential for the uptake and potency of alkylphosphocholine drugs, edelfosine and miltefosine. J Biol Chem 2003, 278, (38), 36041-50).

The conclusions resulting from prior studies and that are relevant to the present invention can be summarised as follows: i) MT and other alkyl phospholipids are incorporated selectively into inferior eukaryotes and transformed cells; ii) the lethal action of these compounds is associated to a massive intracellular incorporation of said drugs and iii) the mechanisms of resistance to MT that have been identified to date in various types of parasites are exclusively related to the defective accumulation of said drug in said organisms.

Recently, the massive use of MT has been initiated in patients infected with visceral leishmaniasis in India and Nepal, especially in patients infected with strains resistant to treatment with antimonial drugs (Sundar, S.; Chatterjee, M., Visceral leishmaniasis - current therapeutic modalities. Indian J Med Res 2006, 123, (3), 345-52); the same strategy is also used in various countries of South America, in the treatment of cutaneous leishmaniasis (Soto, J.; Soto, P., Miltefosine: oral treatment of leishmaniasis. Expert Rev Anti Infect Ther 2006, 4, (2), 177-85). Due to the difficulties for correct hospital control of the treatment in these environments, the rapid appearance of MT-resistant strains of the parasite is predictable. Experiments in animal models have demonstrated that the virulence of the resistant parasites, due to the deficiency in the MT translocators, is the same as that of the drug-sensitive parasites (Seifert, K.; Perez-Victoria, F. J.; Stettler, M.; Sanchez-Canete, M. P.; Castanys, S.; Gamarro, F.; Croft, S. L., Inactivation of the miltefosine transporter, LdMT, causes miltefosine resistance that is conferred to the amastigote stage of Leishmania donovani and persists in vivo. Int J Antimicrob Agents 2007, 30, (3), 229-35). The availability of a reagent and of a fast and easy to handle procedure, such as the one of the present invention, will allow early detection and interception of the appearance of resistant strains.

On a separate note, the early diagnosis of the presence in the eye of *Acanthamoeba,* an amoebic infection with a particularly high incidence among users of contact lenses with poor hygiene habits, is carried out at present after previously discounting potential fungal and herpes infections, with the ensuing damage caused by such delay, which occasionally leads to the irreversible loss of vision (Hammersmith, K. M., Diagnosis and management of Acanthamoeba keratitis. Curr Opin Ophthalmol 2006, 17, (4), 327-31).

The additional application of the compounds described in the present invention to the quality control of contact lens preservation and cleaning fluids, as well as storage means for transplant corneas, is based on the same principles. Recently, a contamination by *Fusarium* of the preservation fluid of lenses seriously affected a large number of patients, with the resulting financial losses for the manufacturer (Chang, D. C.; Grant, G. B.; O'Donnell, K.; Wannemuehler, K. A.; Noble-Wang, J.; Rao, C. Y.; Jacobson, L. M.; Crowell, C. S.; Sneed, R. S.; Lewis, F. M.; Schaffzin, J. K.; Kainer, M. A.; Genese, C. A.; Alfonso, E. C.; Jones, D. B.; Srinivasan, A.; Fridkin, S. K.; Park, B. J., Multistate outbreak of Fusarium keratitis associated with use of a contact lens solution. JAMA 2006, 296, (8), 953-63; Centers for Disease Control and Prevention (CDC), Update: Fusarium keratitis--United States, 2005-2006. MMWR Morb Mortal Wkly Rep 2006, 55, (20), 563-4).

The populations of free living protozoa constitute sentinel organisms of water quality, as well as environmental contamination (Martin-Cereceda, M.; Perez-Uz, B.; Serrano, S.; Guinea, A., Dynamics of protozoan and metazoan communities in a full scale wastewater treatment plant by rotating biological contactors. Microbiol Res 2001, 156, (3), 225-38), meaning that the detection and display of these microorganisms by means of the compound of the invention would be facilitated following their concentration and incorporation of the fluorescent analogue.

There are prior studies that have synthesised and used fluorescent analogues of alkyl phospholipids:
1.- Prior analogues have been prepared of a similar compound (edelfosine), marked with phenyl tetraene groups, and it has been observed that said marker does not substantially alter the bioactivity of the original drug (Quesada, E.; Delgado, J.; Gajate, C.; Mollinedo, F.; Acuna, A. U.; Amat-Guerri, F., Fluorescent phenylpolyene analogues of the ether phospholipid edelfosine for the selective labelling of cancer cells. J Med Chem 2004, 47, (22), 5333-5).
2.- MT analogues have been prepared with said phenyl tetraene or phenyl triene marker groups that maintain the antiparasitic properties of the original drug and, additionally, specifically incorporate into parasites coming from strains of *Leishmania* sensitive to the drug, but not into those coming from resistant strains (Saugar, J. M.; Delgado, J.; Hornillos, V.; Luque-Ortega, J. R.; Amat-Guerri, F.; Acuna, A. U.; Rivas, L., Synthesis and Biological Evaluation of Fluorescent Leishmanicidal Analogues of Hexadecylphosphocholine (Miltefosine) as Probes of Antiparasite Mechanisms. J Med Chem 2007, 50, (24), 5994-6003).
3.- The fluorescent analogue of pentamidine DB-99 has been used for the early detection of organisms resistant to treatment with arsenical drugs in African tripanosomiasis (Stewart, M. L.; Krishna, S.; Burchmore, R. J.; Brun, R.; de Koning, H. P.; Boykin, D. W.; Tidwell, R. R.; Hall, J. E.; Barrett, M. P., Detection of arsenical drug resistance in Trypanosoma brucei with a simple fluorescence test. Lancet 2005, 366, (9484), 486-7). In *Trypanosoma brucei,* the microorganism causing said infection, the introduction of pentamidine and DB99 inside the parasite is carried out through the same transporter of arsenical drugs. Consequently, the absence or dysfunction of said transporter entails inhibition of the internalisation of pentamidine and DB-99 and, therefore, the inhibition of the fluorescent labelling thereof, as well as the appearance of a phenotype of resistance to arsenical drugs.

### DESCRIPTION OF THE INVENTION

### Brief Description

One aspect of the invention consists of a fluorescent compound useful for the differential identification of microorganisms, hereinafter compound of the invention, which comprises an analogue of an alkyl phospholipid with a fluorescent group of high photostability that emits in the visible spectrum wavelength zone.

As used in the present invention, the term "alkyl phospholipid analogue" relates to a compound obtained through synthesis and whose structure is similar to that of an alkyl phospholipid. As used in the present invention, the term "alkyl phospholipid" relates to a compound that includes in its structure a lipophilic residue, formed by linear or cyclical combinations of carbon and hydrogen atoms (alkyl part) joined to a hydrophilic residue, formed by an esterified or free phosphate group (phospholipid). Among alkyl phospholipids can be found, by way of illustration and without limiting the scope of the invention: hexadecylphosphocholine (miltefosine, MT), 1-*O*-octadecyl-2-*O-*methyl-*sn*-glycero-3-phosphocholine (edelfosine, EF) and 1-S-hexadecyl-2-methoxymethyl-*rac*-glycero-3-phosphocholine (ilmofosine, IM). Examples of analogues of alkyl phospholipids are, by way of illustration and without limiting the scope of the invention, *everything-*(*E*)*-*13-phenyltrideca-6,8,10,12-tetraenylphosphocholine and *everything*-(*E*)-13-phenyltrideca-8,1 0,1 2-triene-6-inilphosphocholine.

As used in the present invention, the term "microorganism" relates to any protozoa, amoeba, fungi, yeast and bacteria, belonging, by way of illustration and without limiting the scope of the invention, to the following group: *Leishmania sp, Trypanosoma brucei, Trypanosoma cruzi, Trichomonas sp., Entamoeba histolytica, Acanthamoeba sp., Naegleria sp., Streptococcus pneumoniae, Fusarium sp., Tetrahymena pyriformis, Balamuthia*

As used in the present invention, the term "fluorescent group" relates to a fluorescent group of high photostability that emits in the visible spectrum wavelength zone, among which can be found, by way of illustration and without limiting the scope of the invention: boron dipyrromethenes, xanthenes, rodamines, cyanines, oxacines, porphyrins, coumarines and polycyclic hydrocarbons, with or without substituents.

One more particular aspect of the invention consists of a compound of the invention wherein the alkyl phospholipid analogue is miltefosine (MT), edelfosine (EF) or ilmofosine (IM).

One more particular aspect of the invention consists of a compound of the invention wherein the alkyl phospholipid analogue is an analogue of miltefosine (MT), edelfosine (EF) or ilmofosine (IM).

One particular aspect of the invention consists of a compound of the invention wherein the fluorescent group is free or substituted boron dipyrromethene (BDP).

One particular aspect of the invention consists of a compound of the invention wherein the alkyl phospholipid derivative is miltefosine and the fluorescent group is free or substituted BDP.

One more particular aspect of the invention consists of the compound of the invention BDP-MT or Et-BDP-MT.

A particular aspect of the invention consists of a process of obtaining the compound of the invention comprising the following steps:
i) obtaining the corresponding α-H-pyrrole substituted from a dicetel alcohol and ethyl acetoacetate,
ii) condensation of α-H-pyrrole with the appropriate α- formyl pyrrole,
iii) reaction of dipyrromethane thus formed with boron trifluoride etherate, introduction of the phosphocholine group.

Another particular aspect of the invention consists of the use of a compound of the invention in the manufacture of a useful pharmaceutical composition as a method of identifying microorganisms.

Another particular aspect of the invention consists of the diagnostic pharmaceutical composition, hereinafter diagnostic pharmaceutical composition of the invention, comprising a compound of the invention, for example, the compound BDP-MT or Et-BDP-MT.

Finally, another particular aspect of the invention is the use of diagnostic pharmaceutical composition of the invention, hereinafter use of the pharmaceutical composition of the invention, in a method of identifying microorganisms.

Another particular aspect of the invention consists of the use of the diagnostic pharmaceutical composition of the invention in a ex vivo diagnostic procedure from human or veterinary biological samples of carriers of infectious diseases belonging, for illustrative purposes and without limiting the scope of the invention, to the following group: ocular keratitis, leishmaniasis, African and American trypanosomiasis, Streptococcus pneumoniae pneumonia, amoebiasis, trichomoniasis and mycosis.

Another more particular embodiment consists of the use of the pharmaceutical composition of the invention wherein the infectious disease is caused by a microorganism belonging, for illustrative purposes and without limiting the scope of the invention, to the following group: *Leishmania sp, Trypanosoma brucei, Trypanosoma cruzi, Trichomonas sp., Entamoeba histolytica, Acanthamoeba sp., Naegleria sp., Streptococcus pneumoniae, Fusarium sp., Tetrahymena pyriformis, Balamuthia.*

Another particular embodiment of the invention consists of the use of the diagnostic pharmaceutical composition of the invention wherein the diagnostic procedure is performed on a biological sample of ocular origin, for example, obtained by corneal scraping.

Another more particular aspect of the invention consists of the use the of diagnostic pharmaceutical composition of the invention wherein the procedure corresponds with the identification of microorganisms in non-human and non-animal samples, for example in water, artificial eyes, contact lenses, contact lens cleaning liquid or corneal preservation media.

### DETAILED DESCRIPTION

The present invention is based on that the inventors have found that bioactive analogues of alkyl phospholipids, such as the compounds BDP-MT o Et-BDP-MT, which contain fluorescent groups in their structure with a high photostability emitting in the visible spectrum wavelength zone of 400-700 nm (Slavik, J., Fluorescent Probes in Cellular and Molecular Biology 1ed., CRC Press: Boca Raton, FL, 2004, p 320), are easily incorporated into microorganisms, and that this incorporation is detectable with conventional fluorescence microscopes. Thus, an expert in visual diagnosis could easily identify the presence of microorganisms, e.g. fungi, protozoa, and even some bacteria, such as Streptococcus pneumoniae, etc. capable of incorporating such compounds. Moreover, once identified the species, the expert could deduce the possible resistance to treatment with alkyl phospholipids, which relate directly to a poor incorporation of the compound and a phenotype of resistance to the cytotoxic action of. To this end it suffices to add the fluorescent analogue to the medium or tissue in which the presence of microorganisms is suspected. For example, by direct application on the cornea of the pharmaceutical composition comprising the compound. In the affirmative case, these organisms are quickly labelled with the fluorescent analogue, allowing visual identification by the usual methods of microscopic observation. Furthermore, if the organisms contain the specific carrier of the original MT drug, the rapid appearance of fluorescence is observed in their interior. On the contrary, if the organism is resistant to the MT drug under the same conditions no fluorescence is observed inside the parasite. This provides a rapid and simple diagnostic method of the presence of these microorganisms and / or their resistance to the MT drug.

More specifically, and as a particular example of the invention, a series of fluorescent analogues of MT have been biologically synthesized and tested; these were characterized in that they incorporate a fluorescent group boron dipyrromethane (BDP) to the aliphatic chain of the compound (see examples). When such emitting analogues are added to a physiological environment in which Acanthamoeba trophozoites and fungal hyphae and conidia are present, they quickly incorporate in both types of organisms, allowing proper viewing and subsequent individual identification based on morphological characteristics. This method would facilitate the differential diagnosis of microorganisms in eyes, as it is possible to apply these compounds to that body in the form of eye drops, for direct observation. Also, the method outlined is applicable to the diagnosis of the presence of such organisms in samples from biological tissues or fluids, e.g. corneal scrapings, or for the analysis of corneal transplant preservation liquids or preservation and cleaning liquids for contact lens.

Shown hereunder are possible applications of these compounds, based on studies conducted to date and summarized in the state of the art:
1.- Rapid and reliable diagnosis of eye diseases, ocular keratitis caused by fungal or amoebic infections (Acanthamoeba sp.)
2.-Rapid and reliable diagnosis of possible contamination in eye prostheses (contact lenses, etc..) contact lenses cleaning liquid or corneal preservation media, based on the detection of the presence of pathogenic organisms in said media.
3.-Rapid diagnosis of patients and animals (dogs) infected with strains of Leishmania which are resistant to miltefosine antiparasite drugs, based on the detection of resistant parasites, both in physiological fluids, including peripheral blood, and in samples and tissue biopsies from said patients.
4.-Rapid diagnosis of the degree of environmental health, based on the identification of free-living protozoa in aqueous media of environmental relevance.
5.-Research at a macroscopic and molecular level, using bioimaging techniques, with similar pharmacological and biological properties of miltefosine and alkyl phospholipids, both in cell studies and animal experiments, based on differential identification of the fluorescence of these analogues in different organs and tissues of experimental animals.

Therefore, one aspect of the invention consists of a fluorescent compound which is useful for differential identification of microorganisms, hereinafter compound of the invention, comprising an analogue of alkyl phospholipids with a fluorescent group with high photostability emitting in the visible spectrum wavelength zone.

As used herein the term "alkyl phospholipid analogue" refers to a compound obtained by synthesis and whose structure resembles that of an alkyl phospholipid. As used herein the term "alkyl phospholipid" refers to a compound that includes within its structure a lipophilic remainder consisting of linear or cyclic combinations of carbon and hydrogen atoms (alkyl portion) attached to a hydrophilic remainder consisting of an esterified or free phosphate group (phospholipid). Alkyl phospholipids include, by way of illustration and without limiting the scope of the invention: hexadecylphosphocholine (miltefosine, MT), 1-O-octadecyl-2-O-methyl-sn-glycero-3-phosphocholine (edelfosine, EF) and 1-S-hexadecyl-2-methoxymethyl-rac-glycero-3-phosphocholine (ilmofosine, IM). Examples of alkyl phospholipid analogues are, by way of illustration and without limiting the scope of the invention, all-(E)-13-phenyltridec-6,8,10,12-tetraenilphosphocholine and all-(E)-13-phenyltridec 8,10,12-trien-6- inilphosphocholine

As used herein the term "microorganism" refers to any protozoa, amoeba, fungus, yeast and bacteria, belonging, by way of illustration and without limiting the scope of the invention, to the following group: *Leishmania sp, Trypanosoma brucei, Trypanosoma cruzi, Trichomonas sp., Entamoeba histolytica, Acanthamoeba sp., Naegleria sp., Streptococcus pneumoniae, Fusarium sp., Tetrahymena pyriformis, Balamuthia.*

As used herein the term "fluorescent group" refers to a fluorescent group with a high photostability emitting in the visible spectrum wavelength zone, comprising, by way of illustration and without limiting the scope of the invention: boron dipyrromethanes, xanthenes, rhodamines, cyanines, oxacin, porphyrins and polycyclic coumarins and hydrocarbons, with or without substituents.

A more particular aspect of the invention consists of a compound of the invention wherein the alkyl phospholipid analogue is miltefosine (MT), edelfosine (EF) or ilmofosine (IM).

A more particular aspect of the invention consists of a compound of the invention wherein the alkyl phospholipid analogue is an analogue of miltefosine (MT), edelfosine (EF) or ilmofosine (IM).

A particular aspect of the invention consists of a compound of the invention wherein the fluorescent group is free or substituted boron dipyrromethane (BDP).

A particular aspect of the invention consists of a compound of the invention wherein the derivative of alkyl phospholipid is miltefosine and the fluorescent group is BDP free or substituted.

A more particular aspect of the invention consists of the compound of the invention BDP-MT or Et-BDP-MT.

A particular aspect of the invention consists of a method of obtaining the compound of the invention comprising the following steps:
i) obtaining the corresponding α-H-pyrrole substituted from a dicetel alcohol and ethyl acetoacetate,
ii) condensation of α-H-pyrrole with the appropriate α- formyl pyrrole,
iii) reaction of dipyrromethane thus formed with boron trifluoride etherate,
iv) introduction of the phosphocholine group.

Another particular aspect of the invention consists of the use of a compound of the invention in the manufacture of a pharmaceutical composition which is useful for a microorganism identification method.

Another particular aspect of the invention consists of the diagnostic pharmaceutical composition, hereinafter diagnostic pharmaceutical composition of the invention, comprising a compound of the invention, for example, the compound BDP-MT or Et-BDP-MT.

Finally, one particular aspect of the invention consists of the use of the diagnostic pharmaceutical composition of the invention, hereinafter use of the pharmaceutical composition of the invention, in a microorganism identification method.

Another more particular aspect of the invention is the use of diagnostic pharmaceutical composition of the invention in an ex vivo diagnostic process of biological samples from human or veterinary carriers of infectious diseases belonging, by way of illustration and without limiting the scope of the invention, the following group: ocular keratitis, leishmaniasis, African and American trypanosomiasis, Streptococcus pneumoniae pneumonia, amoebiasis, trichomoniasis and mycosis.

Another more particular embodiment consists of the use of the pharmaceutical composition of the invention wherein the infectious disease is caused by a microorganism belonging, by way of illustration and without limiting the scope of the invention, the following group: *Leishmania sp, Trypanosoma brucei, Trypanosoma cruzi, Trichomonas sp., Entamoeba histolytica, Acanthamoeba sp., Naegleria sp., Streptococcus pneumoniae, Fusarium sp., Tetrahymena pyriformis, Balamuthia.*

Another more particular embodiment of the invention consists of the use of diagnostic pharmaceutical composition of the invention wherein the diagnostic procedure is performed on a biological sample of ocular origin, for example, obtained by corneal scraping.

Another more particular aspect of the invention consists of the use of diagnostic pharmaceutical composition of the invention wherein the procedure corresponds with the identification of microorganisms in non-human and non-animal samples, for example in water, artificial eyes, contact lenses, cleaning liquid for contact lenses or corneal preservation media

### DESCRIPTION OF THE FIGURES

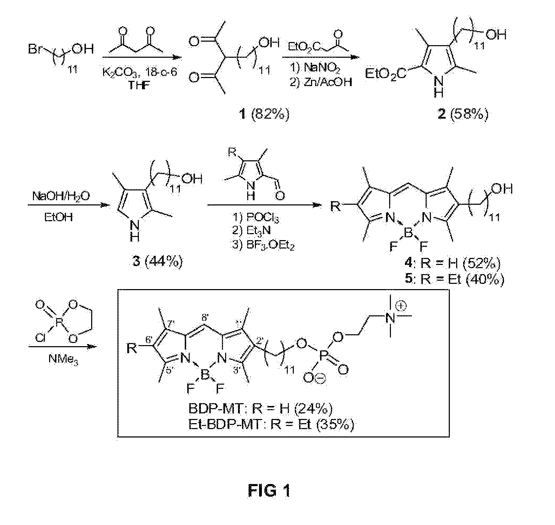

**Figure 1** **.- Synthesis of fluorescent analogues of BDP-MT and Et-BDP-MT, through the intermediates 1 to 5.**

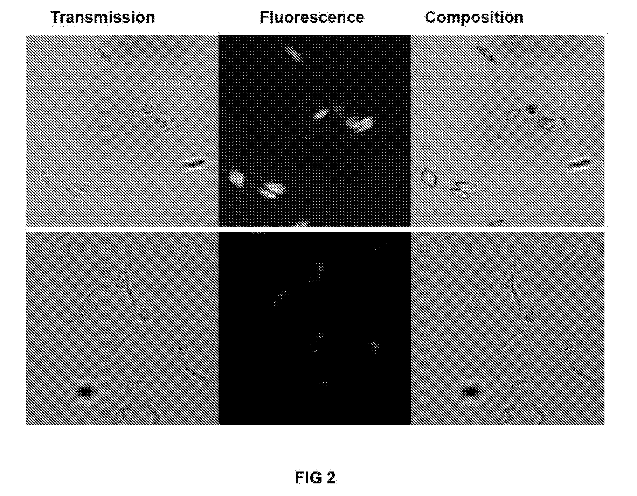

**Figure 2****.-Differential detection of resistance to miltefosine in promastigotes of *L.donovani* (strain MHOM/ET/67/L82).** Promastigotes of L. *Donovani,* being sensitive (WT) (top row) or resistant to MT (R40) (bottom row) were incubated with BDP-MT 7.5 µM for 2 h at 26 °C, then washed with bovine serum albumin (BSA) three times, and examined by confocal microscopy using 488 nm as excitation length and 502-555 nm as reading environment of the emission.

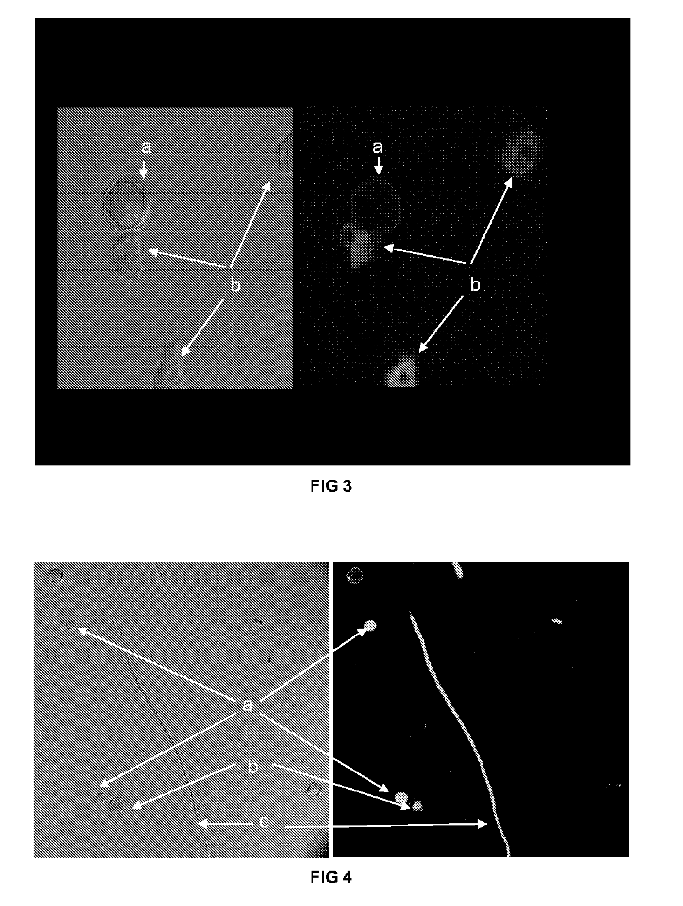

**Figure 3** **.- Differential staining of two differential forms of *Acanthamoeba castellanii* by BDP-MT.** Cysts (a) and trophozoites (b) of *A. castellanii* were incubated at 32°C with 5 µM BDP-MT for 15 min, then washed with a solution of BSA (10 mg / mL) and observed under a confocal microscope, using 488 nm as excitation wavelength and detecting the emission in the interval 502-555 nm.
**Figure 4** **.- Simultaneous differential staining by BDP-MT of Fusarium and the two forms of *Acanthamoeba castellanii.*** Trophozoites (a) and cysts (b) of *A. castellanii* in combination with hyphae of Fusarium sp. (c) were incubated 15 minutes with 5 µM BDP-MT and washed with BSA. They were observed under a confocal microscope using 488nm as excitation wavelength and detecting the emission in the interval 502-555 nm.

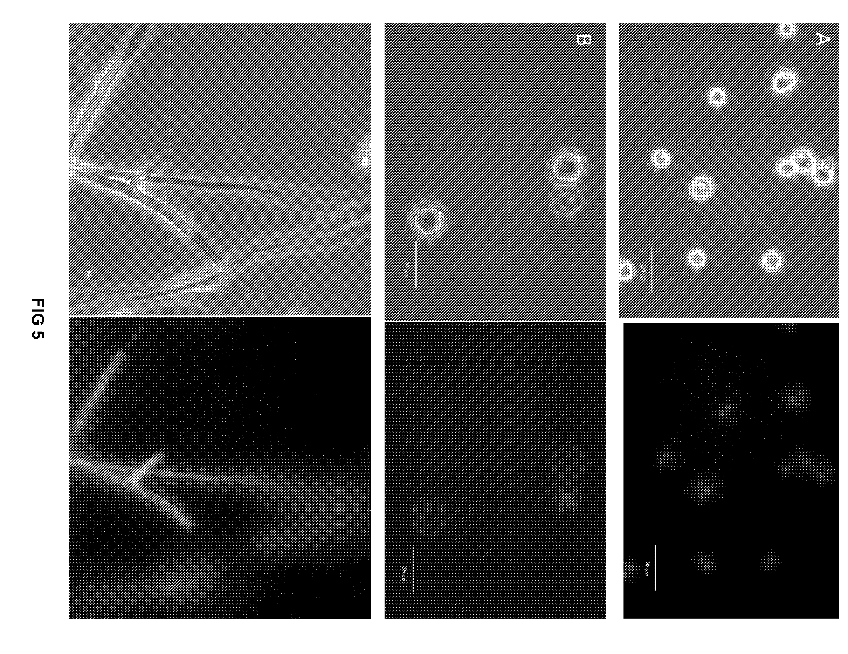

**Figure 5****.- Simultaneous differential staining by Et-BDP-MT of *Fusarium* and the two forms of** ***Acanthamoeba castellanii.** A. castellanii* trophozoites (a) and cysts (b) together with *Fusarium sp* hyphae (c) were incubated for 15 minutes at 30°C with 5 µM Et-BDP-MT and they were washed with BSA. The fluorescence was observed using an epifluorescence microscope with CCD camera, and 480-520 nm emission filter.

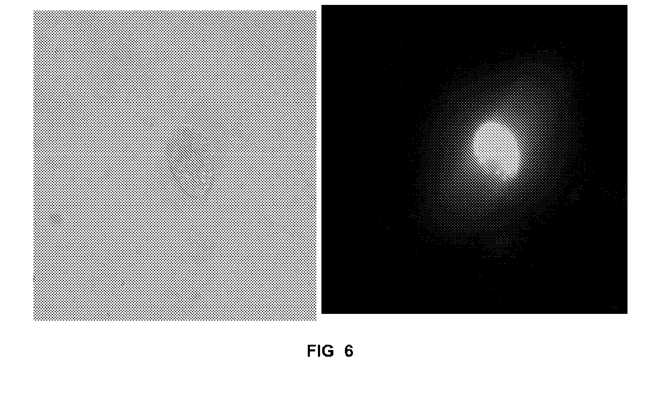

**Figure 6****.- Staining by BDP-MT of *Tetrahymena pyriformis,* as free-living protozoa.** *Tetrahymena pyriformis* was incubated with 5 µM BDP-MT for 4 h, and it was washed and fixed with 2% paraformaldehyde, to avoid movement during the image acquisition process. The cells were observed in a confocal microscope using 488 nm as excitation wavelength and detecting the emission in the 502-555 nm interval.

### EXAMPLES OF EMBODIMENT

### Examples 1.- Obtaining two compounds analogous to miltefosine that incorporated in their structure fluorescent groups in the visible zone: BDP-MT and Et-BSP-MT.

These two fluorescent analogues with the chromophore borodipyrromethane (BDP) are obtained by a similar two-step process, starting from the corresponding pyrroles (Figure 1). Thus, the α-H pyrrole 3 is condensed with 2- formyl-3,5-dimethyl-1 /-/-pyrrole or with 2-formyl-3,5-dimethyl-4-ethyl-1 H-pyrrole in the presence of phosphorus oxychloride (MacDonald's reaction). The corresponding dipyrromethanes formed are converted *in situ* in the respective BDP colorants substituted **4** and **5** by reaction with boron trifluoride etherate in the presence of triethylamine. In a last synthetic step, the phosphocholine group is introduced by reaction with 2-chloro-1 ,3,2-dioxaphospha!ene-2-oxide in the presence of trimethylamine. The precursors are obtained as follows: diketoalcohol 1 is achieved by monoalkylation of acetylacetone with 11- bromo-1-undecano! in acetone, in the presence of potassium carbonate and 18-crown-6 ether; ethoxycarbonylpyrrol 2 is prepared in two steps: 1) ethyl acetoacetate is treated with sodium nitrate, and 2) the hydroxy-imine compound obtained in reduced with zinc/acetic acid (Johnson-Knorr synthesis) in the presence of 1. α-H pyrrole 3 is obtained by decarboxylation of 2 by treatment with sodium hydroxide in water/ethanol. 2-Formyl-3,5-dímethyl-1/-/-pyrrole is a commercial product, whilst its homologue 2-formyl-4-ethyl-3,5-dimethyl- 1 /-/-pyrrole is obtained by formylation of 2,5-dimethyl-3-ethyl-1H-pyrrole (kryptopyrrole) with phosphorus oxychloride in dimethylformamide.

### Example 2.- Diagnosis of the presence of parasites of the genus Leishmania sensitive to or resistant to the drug miltefosine, by means of incorporating the fluorescent analogue BDP-MT.

The promastigotes of *L. Donovani*, MHOM/ET/67/L82 strain, and its strain resistant to miltefosine were provided by Prof Simon Croft (London School of Tropical Hygiene and Medicine, London, UK) and they were cultured in accordance with habitual methods: 26ºC in RPMI supplemented with 10% inactivated fetal bovine serum, gentamicin, penicillin and 2 mM glutamine. The MT resistant strain was grown identically to the MT-sensitive parental, except for the addition of 40 µM MT in the culture medium. Both types of parasites were collected in stationary phase, they were washed with RPMI 1640 medium lacking phenol red, and they were incubated in said medium for 2 hours at 261°C at a density of 2 x 10⁶ parasites/mL to which 7.5 µM BDP- MT was added. They underwent three washes with said medium in the presence of BSA free from fatty acids, at 10 mg/mL, and finally they were detected *in vivo* in a Leica TCS-SP2-AOBS-UV confocal microscope, using 488 nm as excitation wavelength and 502-555 nm as emission reading range.

It can be observed in Figure 2 how the parasites of the sensitive strain show an intense intracellular fluorescence, whist those from the resistant strain show weak fluorescence of the BDP-MT marker analogue.

### Example 3.- Differential diagnosis of ocular parasites by BDP-MT

### 3.1. - Acanthamoeba castellanii trophozoites and cysts

*A castellanii* trophozoites and cysts were provided by Dr Carmen del Aguila, Universidad San Pablo CEU, Madrid. The trophozoites were grown in CDC medium supplemented with fetal bovine serum and they were incubated in the same medium for 15 minutes at a density of 7 x 10⁵ trophozoites at 32ºC with 5 µM BDP-MT. After said incubation, they were washed in the same medium with 10 mg/mL of BSA and they were observed *in vivo* in a Leica TCS-SP2-AOBS-UV confocal microscope, using 488 nm as excitation wavelength and 502-555 nm as emission reading range.

It can be observed in Figure 3 that the *A. caslellanii* trophozoites, which the metabolically active form of the parasite consists of, show an intense intracellular fluorescence marking due to the high incorporation of BDP-MT. In the case of the cysts, said figure shows that the fluorescent staining is concentrated on the surface. As this experiment was carried out with live organisms, there are small differences in position between the consecutive transmission and fluorescence images, due to mobility of the trophozoites.

### 3.2.- Simultaneous fluorescence marking of A castellanii cysts and trophozoites and Fusarium hyphae.

The hyphae of the *Fusarium* fungus were kept in agarose with Saboroud medium. The cell expansion was carried out at 32ºC in RPMI 1640 medium with antibiotics and without supplementing with fetal bovine serum. After washing, the hyphae were resuspended for 15 minutes at 32°C in identical medium without phenol red and in the presence of BDP-MT 5 µM. Next, they were washed three times with BSA and were observed in vivo in a Leica TCS-SP2-AOBS-UV confocal microscope, using 488 nm as excitation wavelength and 502-555 nm as emission reading range. The fluorescent images observed after incorporation of BDP-MT are very different for the three forms: hyphae, trophozoites and cysts, in the case of the hyphae, their characteristic elongated morphology is clearly appreciated, whilst in the case of *A. castellanii* trophozoites, of considerably smaller size, irregular forms are observed; in ambos cases the intracellular incorporation of the fluorescent analogue is observed. In the case of the *A. castellanii*, cysts, essentially spherical in shape, the fluorescent marking is only observed on the organism's surface, as already commented in the previous example.

### Example 4.- Diagnosis of the presence of pathogenic microorganisms by means of the fluorescent analogue Et-BDP-MT.

The method of fluorescent marking of pathogenic organisms demonstrated above, using the compound BDP-MT, can also be carried out using other analogues, wherein, altering the structure of the fluorescent group, it achieves that the excitation wavelength and that of emission appear in other different zones of the visible spectrum. To illustrate this extension in the present example, the ET-BDP-MT analogue is used, wherein the excitation and emission occur at wavelengths displaced towards the red, in comparison with the BDP-MT analogue. The three panels of Figure 5 contained representative examples of organisms stained with Et-BDP-MT, and there show *Acanthamoeba castellanii* trophozoites (A), *Acanthamoeba castellanii* cysts (B), and *Fusarium* hyphae (C). In all cases, the microorganisms were marked maintaining them in the presence of the Et-BDP-MT analogue, 5 µM, for 30 minutes. They were then washed with medium containing 10 mg/mL BSA and they were photographed using a CCD Digital Leica DFC350FX camera attached to a Zeiss epifluorescence microscope with a 480-520 nm emission filter.

In all cases a fluorescence pattern was observed very similar to that obtained for BDP-MT; the *A caslellanii* trophozoites with intracellular marking, the cysts of the same microorganisms, not damaged with faint surface marking, and *Fusarium* hyphae marking, which demonstrates that the data obtained for BDP-MT are easily extrapolated to other structurally similar compounds.

### Example 5.- Fluorescent marking of the free-living protozoan Tetrahymena with BDP-MT.

Cells of the *Tetrahymena pyriformis* protozoan were multiplied in the Protease Peptone Glucose Medium, and they were collected by centrifugation at a density of 10⁴ cells/mL. Next, they were incubated with BDP-MT 5 µM for 4 h, they were washed and fixed with 2% paraformaldehyde for 30 minutes and they were observed with a Leica TCS-SP2-AOBS- UV confocal microscope, using 488 nm as excitation wavelength and 502-555 nm as emission reading range. As can be observed in Figure 6, the microorganism incorporates a great quantify of fluorescent compound, which enables its identification and location, even at very low cell concentrations.

## Claims

1. Fluorescent compound useful for the differential identification of microorganisms, **characterised in that** it comprises an alkyl phospholipid derivative and a highly photostable fluorescent group emitting in the visible spectrum wavelength zone.

2. Fluorescent compound according to claim 1, **characterised in that** the alkyl phospholipid analogue is miltefosine (MT), edelfosine (EF) or ilmofosine (IM).

3. Fluorescent compound according to claim 1, **characterised in that** the alkyl phospholipid analogue is an analogue of miltefosine (MT), edelfosine (EF) or ilmofosine (IM).

4. Fluorescent compound according to claim 1, **characterised in that** the fluorescent group is a highly photostable fluorescent group emitting in the visible spectrum wavelength zone, including, by way of illustration and without limiting the scope of the invention: borodipyrromethanes, xanthenes, rhodamines, cyanines, oxazines, porfirins, coumarins and polycyclic hydrocarbons, with or without substituents.

5. Fluorescent compound according to claims 1 to 4, **characterised in that** the fluorescent group is borodipyrromethane (BDP), free or substituted, **characterised in that** it has the general formula: 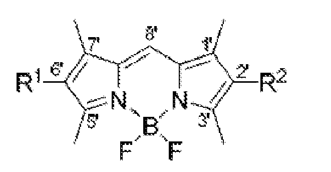 wherein:
R¹ = H, alkyl (linear, non-linear, free or substituted), aryl or heteroaryl (free or substituted), and R² = alkyl-, alkenyl- or alkinyl phospholipid.

6. Fluorescent compound according to claim 5 **characterised in that** R¹ is a hydrogen or an ethyl.

7. Fluorescent compound according to claims 1 and 2, **characterised in that** the alkyl phospholipid analogue is miltefosine and the fluorescent group is BDP free or substituted, **characterised in that** it has the general formula: 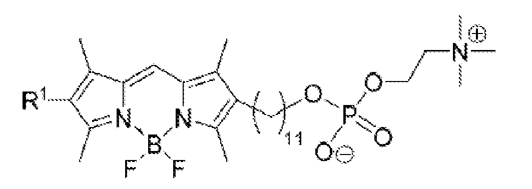 wherein:
R¹ = H, alkyl (linear, non-linear, free or substituted), aryl or heteroaryl (free or substituted).

8. Fluorescent compound according to claim 7, **characterised in that** R¹ is a hydrogen (BDP-MT) or a radical ethyl (Et-BDP-MT compound).

9. Obtaining process of a fluorescent compound **characterised in that** it comprises the following stages: i) obtaining the corresponding α-H-pyrrole substituted from a diketoalcohol and ethyl acetoacetate, ii) condensation of the α-H-pyrrole with the suitable α-formylpyrrol, iii) reaction of the dipyrromethane thus formed with boron trifluoride etherate, iv) introduction of the phosphocholine group.

10. Use of a fluorescent compound according to claims 1 to 8 in producing a pharmaceutical composition useful for a microorganism identification process.

11. Diagnostic pharmaceutical composition **characterised in that** it comprises a compound according to claims 1 to 8.

12. Diagnostic pharmaceutical composition according to claim 11, **characterised in that** it comprises at least one of the following compounds: BDP-MT and Et-BDP-MT.

13. Use of the diagnostic pharmaceutical composition according to claims 11 and 12, in a microorganism identification process.

14. Use of the diagnostic pharmaceutical composition according to claim 13, in an *ex vivo* diagnostic process from human or veterinarian biological samples of individuals carrying infectious diseases, belonging, by way of illustration and without limiting the scope of the invention, to the following group: ocular keratitis, leishmaniasis, African and American typanosomiasis, pneumonia due to *Streptococcus pneumoniae,* amoebiasis, trichomoniasis and mycosis.

15. Use of the diagnostic pharmaceutical composition according to claim 14, **characterised in that** the infectious disease is caused by a microorganism belonging to the following group: *Leishmania sp, Trypanosoma brucei, Trypanosoma cruzi Trichomonas sp., Entamoeba histolytica, Acanthamoeba sp., Naegleria sp., Streptococcus pneumoniae, Fusarium sp., Tetrahymena pyriformis, Balamuthia.*

16. Use of the diagnostic pharmaceutical composition according to claim 14, in a diagnosis process on a sample of ocular origin, for example, obtained by corneal scraping.

17. Use of the diagnostic pharmaceutical composition according to claim 13, in a microorganism identification process in non-human and non-animal samples, for example, in waters, eye prostheses, contact lenses, contact lens cleaning liquids or means for preserving corneas.
